(19) Europäisches Patentamt
European Patent Office
Office européen des brevets

(11) Veröffentlichungsnummer: **0 016 214**
**B1**

(12) **EUROPÄISCHE PATENTSCHRIFT**

(45) Veröffentlichungstag der Patentschrift: **03.02.82**

(51) Int. Cl.³: **C 07 C 1/00**, C 07 C 9/04, C 10 J 3/20, C 10 J 3/56

(21) Anmeldenummer: **79901101.0**

(22) Anmeldetag: **21.08.79**

(86) Internationale Anmeldenummer: **PCT/DE 79/00091**

(87) Internationale Veröffentlichungsnummer: **WO WO/80004 (20.03.80 Gazettee 80/06)**

# (54) ANLAGE ZUR KOHLEVERGASUNG MIT HYDRIERENDEM UND WASSERDAMPFVERGASER.

(30) Priorität: **31.08.78 DE 2837906**

(43) Veröffentlichungstag der Anmeldung: **01.10.80 (Patentblatt 80/20)**

(45) Bekanntmachung des Hinweises auf die Patenterteilung: **03.02.82 Patentblatt 82/05**

(84) Benannte Vertragsstaaten: **AT CH FR GB LU NL SE**

(56) Entgegenhaltungen:
**DE - A - 2 403 933**
**DE - A - 2 553 506**
**DE - A - 2 609 320**
**DE - A - 2 704 465**

(73) Patentinhaber: **GHT Gesellschaft für Hochtemperaturreaktor-Technik mbH
Friedrich-Ebert-Strasse
D-5060 Bergisch-Gladbach 1 (DE)**

(72) Erfinder: **FISCHER, Reimer
Lückerather Weg 71
D-5060 Bergisch Gladbach 2 (DE)**
Erfinder: **JGER, Walter
Schulweg 33
D-5250 Engelskirchen (DE)**

(74) Vertreter: **Mehl, Ernst, Dipl.-Ing.
Postfach 22 01 76
D-8000 München 22 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

EP 0 016 214 B1

# Anlage zur Kohlevergasung mit hydrierendem und Wasserdampfvergaser

Die vorliegende Erfindung betrifft eine Anlage zur Erzeugung von Methan mit den im Oberbegriff des ersten Anspruchs genannten Merkmalen :

Mehrstufige Kohlevergasungsverfahren sind bekannt, z.B. aus L.J. Anastasia, W.G. Blair : « Clean Fuels from Coal », Symposium II Paper, IGT Chicago 1975, S. 171-193 (sog. Hygas-Verfahren) und aus R.F. Detman : « Stand des OCR/AGA Kohlevergasungsprogrammes » in Gas Wärme International 23 (1974) s. 301-303 (sog. Bigas-Verfahren). Die Kohle wird in einer ersten Stufe hydrierend vergast, wobei die durch den exothermen Prozeß der Mehanisierung freigesetzte Wärme genutzt wird und der dazu erforderliche Wasserstoff in einer zweiten Stufe durch Wasserdampfvergasung erzeugt wird ; ein endothermer Vorgang, der durch Verbrennung eines Teils des eingebrachten Kohlenstoffes in Gang gehalten wird.

Aus der DE-A-26 09 320 ist eine Anordnung bekannt, bei der ein hydrierender und ein Wasserdampfvergaser in einem gemeinsamen Gehäuse übereinander angeordnet sind, doch mit einer zwischengeschalteten Druckschleuse, die einen erhöhten Aufwand bedeutet und eine kontinuierliche Zufuhr von Wasserstoff aus dem Wasserdampfvergaser in den hydrierenden Vergaser verhindert ; das heiße Produktgas aus ersterem wird abgekühlt in einen Konverter geleitet. In einer von der Anmelderin in der DE-A-25 53 506 vorgeschlagenen Anlage zur Kohlevergasung wird der hydrierende Vergaser ausschließlich mit Wasserstoff aus einer Gaszerlegung versorgt, so daß eine Optimierung des Wasserstoffgehaltes in demselben schwierig ist.

Wird der hydrierende Vergaser dagegen nur mit dem Produktgas des Wasserdampfvergaser beschickt, ist der Vergasungsgrad in ersterem wegen des verhältnismäßig geringen Wasserstoffpartialdruckes in diesem Gas nur gering. Der kohlenstoffreiche Restkoks kann auch in einem nachgeschalteten Wasserdampfvergaser nicht restlos genutzt werden, erst recht nicht, wenn wie bisher vorgeschlagen, die Wasserdampfvergasung nur in einer Stufe erfolgt. Eine Vergasung in einem Festbett macht darüber hinaus auch ein Abkühlen und Brikettieren des Restkokses notwendig, was zu unerwünschten Wärmeverlusten führt.

Aufgabe der vorliegenden Erfindung ist eine verbesserte Anlage zur Kohlevergasung, in der der in der Rohkohle enthaltene Kohlenstoff möglichst vollständig genutzt wird und die Wärmeverluste gering gehalten werden. Ferner solche Ausgestaltungen der Erfindung, die die zur Aufrechterhaltung des Prozesses erforderliche Wärme möglichst unter Verzicht auf Verbrennung der Kohle erzeugen.

Zur Lösung dieser Aufgabe wird eine Anlage mit den im kennzeichnenden Teil des ersten Anspruchs genannten Merkmalen vorgeschlagen. Dadurch, daß dem Produktgas des Wasserdampfvergasers der Wasserstoff aus der Gaszerlegung beigemischt wird, steigt der Wasserstoffpartialdruck im hydrierenden Vergaser so weit an, daß bereits hier ein guter Vergasungsgrad erreicht wird. Die direkte Weiterleitung des heißen Restkokses in den Wasserdampfvergaser erspart Wärmeverluste. Das Problem einer nutzbringenden Verwertung des Restkokses aus dem Wasserdampfvergaser entfällt, da die eingebrachte Kohle bis auf einen mehr oder weniger geringen Aschenrückstand vollständig vergast ist.

Die einzelnen chemischen Reaktionen, die im Wasserdampfvergaser vor sich gehen, werden dabei zweckmäßigerweise auf getrennte Wirbelstufen verlegt. Die Anzahl der Stufen richtet sich dabei nach der Reaktionsfähigkeit der eingebrachten Rohkohle (für Braunkohle weniger, für Steinkohle dagegen mehr).

Die erforderliche Wärmezufuhr an den Wasserdampfvergaser kann auf die im zweiten Anspruch vorgeschlagene konventionelle Art erfolgen.

Es ist aber auch die im dritten Anspruch vorgeschlagene Alternativlösung möglich ; im Wasserdampfvergaser sind beispielsweise Heizschlangen angebracht, in denen ein Heizfluid ziruliert.

Wenn, wie z.B. in Deutschland, die Rohkohle verhältnismäßig kostspielig ist, kann die im vierten Anspruch genannte Lösung vorteilhaft sein, in der die nötige Wärme beispielsweise durch einen Hochtemperaturreaktor bereitgestellt wird, dessen Kühlgas den Wasserdampfvergaser aufheizt ; der eingebrachte Kohlenstoff fällt dann zu einem höheren Anteil in Form des gewünschten Methans an.

Wenn das aus dem hydrierenden Vergaser austretende Gasgemisch ohnehin, z.B. zum Zwecke der Reinigung, abgekühlt werden soll, kann es auch entsprechend der im fünften Anspruch vorgeschlagenen Lösung zur Beheizung des Wasserdampfvergasers herangezogen werden. Die im siebten und achten Anspruch vorgeschlagenen Lösungen sind Alternativen zur Lösung der dann auftretenden Ungleichgewichte in der Material- und/oder Wärmebilanz der Anlage.

Der größte Teil des Wärmeinhaltes des dem Wasserdampfvergaser zuzuführenden Dampfes wird von der Verdampfungswärme des Wassers gebildet. Bei Berücksichtigung der oben zum vierten Anspruch gemachten Annahmen ist es gemäß dem sechsten Anspruch dann vorteilhaft, die Dampferzeugung ebenfalls niklear zu betreiben, wozu sich z.B. auch ein Leichtwasserreaktor eignet.

Zwei beispielhafte Ausführungsformen der Erfindung sind in der Zeichnung dargestellt, wobei beide Figuren in großen Teilen miteinander identische Schaltbilder der Anlage zeigen, die daher auch zusammen beschrieben werden.

Die Rohkohle wird zunächst in einer vorbereiteten Stufe 1 behandelt, z.B. getrocknet und dann einem hydrierenden Vergaser 3 zugeleitet, der mit einem Wasserdampfvergaser 4 eine bauliche Einheit 2 bildet. Das durch die Reaktion eines Teils des in der Kohle enthaltenen Kohlenstoffes mit dem (wie weiter unten näher beschrieben) zugeführten Wasserstoff

$$C + 2H_2 = CH_4$$

gebildete Methan wird zusammen mit anderen Gasen in einem ersten Wärmetauscher 5 abgekühlt und dann auf der Primärseite eines Regenerativwärmetauschers 6 in eine Waschvorrichtung 7 geleitet, in der das Gas von Feststoffpartikeln, Teer usw. befreit wird, die schließlich über einen Abscheider 8 entfernt werden. Das Gas fließt dann über die Sekundärseite des Regenerativwärmetauschers 6 und unter Zusatz des anderweitig in der Anlage abgeschiedenen Kohlenmonoxids in einen Konverter 9, in dem dieses größtenteils entsprechend der Reaktion

$$CO + H_2O = H_2 + CO_2$$

umgewandelt wird. Nach erneuter Abkühlung in einem weiteren Wärmetauscher 10 und Entfernung von Kohlendioxyd und Schwefelwasserstoff in einer Reinigungsstufe 11 wird das Gas schließlich in einer weiteren Stufe, z.B. einer Tieftemperaturzerlegung 12 in seine Bestandteile Methan (das die Anlage als sog. SNG verläßt, um einem Verbraucher zugeführt zu werden), Stickstoff (der in die Atmosphäre entweicht), Kohlenmonoxyd (das zum Konverter 9 zurückgeführt wird) und schließlich Wasserstoff zerlegt, der wiederum in den hydrierenden Vergaser 3 zurückgeleitet wird und dort den Wasserstoffpartialdruck im Anströmgas soweit erhöht, daß bereits eine weitgehende Vergasung der Kohle (ca. 50-60 %) eintritt. Das restliche Anströmgas stammt aus dem Wasserdampfvergaser 4, in den der Restkoks aus dem hydrierenden Vergaser unmittelbar und ohne abzukühlen geleitet wird. Der Wasserdampf kann konventionell erzeugt sein oder aber auch (in Fig. 1 nur strichpunktiert angedeutet) aus einem Kernreaktor 13 stammen. In den verschiedenen (hier drei) hintereinandergeschalteten Stufen des Wasserdampfvergasers läuft zunächst (in Strömungsrichtung des Dampfes und des zugefügten Sauerstoffs gesehen) die stark exotherme Reaktion

$$C + O_2 = CO_2$$

ab, bei der ein Teil des Kohlenstoffs verbrannt wird und die die nötige Wärme für die endotherme Reaktion

$$C + H_2O = CO + H_2$$

liefert, in der der restliche Kohlenstoff umgesetzt wird, so daß über einen Austrag 15 nur noch Asche zu entfernen ist.

In der in Fig. 2 dargestellten Ausführungsform liefert der Kernreaktor 13 nicht nur den Dampf für die Wasserdampfvergasung, sondern auch ein heißes Fluid (z. B. das als Kühlmittel verwendete Helium), das in Rohrschlangen 16 zirkuliert und so die einzelnen Wirbelschichtbetten des Wasserdampfvergasers 4 beheizt. Auf eine Zufuhr von Sauerstoff kann dann verzichtet werden. Die Rohrschlangen 16 können alternativ hierzu (strichpunktiert dargestellt) auch mit heißem Produktgas aus dem hydrierenden Vergaser 3 beheizt werden. Allerdings muß dann entweder erneut, wenn auch in geringerem Ausmaß Sauerstoff zugeführt werden, um wie oben durch Verbrennung eines Teils des Kohlenstoffs die erforderliche Wärme zu erzeugen, oder die Vergasung läuft bei niedrigeren Temperaturen ab und erzeugt weniger Wasserstoff. Ein Teil des erzeugten Methans muß dann in einem Spaltofen 17 unter Wärme- und Dampfzufuhr entsprechend der Reaktion

$$CH_4 + H_2O = 3H_2 + CO$$

gespalten werden und das so erzeugte Gasgemisch dem hydrierenden Vergaser zugeführt werden.

## Ansprüche

1. Anlage zur Vergasung von Kohle zu Methan mit einem hydrierenden Vergaser (3); einem Wasserdampfvergaser (4), in dem der Restkoks aus dem hydrierenden Vergaser vergast wird; einem Konverter (9) zur Umwandlung des im Wasserdampfvergaser erzeugten Kohlenmonoxids unter Zusatz von Wasserdampf und Kohlenmonoxid; und einer Gaszerlegung (12) zum Abscheiden von Methan, Wasserstoff und Kohlenmonoxid, gekennzeichnet durch folgende Merkmale:

a) Der Hydrierende Vergaser (3) ist mit einer Wasserstoff führenden Leitung sowohl mit dem Wasserdampfvergaser (4) als auch der Gaszerlegung (12) verbunden.

b) Hydrierender Vergaser (3) und Wasserdampfvergaser (4) sind übereinander angeordnet; der Restkoks aus ersterem fließt unmittelbar in letzteren.

c) Der Wasserdampfvergaser (4) weist mehrere hintereinander geschaltete Wirbelbettstufen auf.

2. Anlage nach Anspruch 1, gekennzeichnet durch folgendes Merkmal:

a) Der Wasserdampfvergaser (4) ist in an sich bekannter Weise durch Verbrennung eines Teils des Restkokses mit gesondert zugeführtem Sauerstoff beheizt.

3. Anlage nach Anspruch 1, gekennzeichnet durch folgendes Merkmal:

a) Der Wasserdampfvergaser (4) ist indirekt mittels eines heißen Fluids beheizt.

4. Anlage nach Anspruch 3, gekennzeichnet durch folgendes Merkmal:

a) Der Wasserdampfvergaser (4) ist mit dem Kühlfluid eines Kernreaktors (13) beheizt.

5. Anlage nach Anspruch 3, gekennzeichnet durch folgendes Merkmal :

a) Der Wasserdampfvergaser (4) ist mit dem Produktgas des hydrierenden Vergasers (3) beheizt.

6. Anlage nach einem oder mehreren der Ansprüche 1-5 gekennzeichnet durch folgendes Merkmal :

a) Sie schließt einen Kernreaktor (13) zur Erzeugung des dem Wasserdampfvergaser (4) zugeführten Wasserdampfes ein.

7. Verfahren zum Betrieb einer Anlage nach Anspruch 5 oder 6, gekennzeichnet durch folgendes Merkmal :

a) Die zum Ausgleich der Wärmebilanz bei ausgeglichener Materialbilanz der Anlage erforderliche Wärme wird durch Verbrennung eines Teils des Restkokses mit gesondert zugeführtem Sauerstoff erzeugt.

8. Verfahren zum Betrieb einer Anlage nach Anspruch 5 oder 6, gekennzeichnet durch folgendes Merkmal :

a) Der zum Ausgleich der Materialbilanz bei ausgeglichener Wärmebilanz der Anlage erforderliche Wasserstoff ist in einem Spaltofen (17) aus einem Teil des Methans erzeugt.

## Claims

1. An installation for the gasification of coal to methane comprising a hydrogenating gasifier (3) ; a steam gasifier (4), in which the residual coke from the hydrogenating gasifier is gasified ; a converter (9) which serves for the conversion of the carbon monoxide produced in the steam gasifier with the addition of steam and carbon monoxide ; and a gas separation unit (12) for the separation of methane, hydrogen and carbon monoxide, characterised by the following features :

a) The hydrogenating gasifier (3) is connected by a hydrogen conveying line both to the steam gasifier (4) and to the gas separation unit (12).

b) The hydrogenating gasifier (3) and steam gasifier (4) are arranged above one another ; the residual coke from the former flows directly into the latter.

c) The steam gasifier (4) comprises a plurality of series-arranged vortex bed stages.

2. An installation as claimed in Claim 1, characterised by the following feature :

a) The steam gasifier (4) is heated in known manner by burning a part of the residual coke with separately supplied oxygen.

3. An installation as claimed in Claim 1, characterised by the following feature :

a) The steam gasifier (4) is indirectly heated by means of a hot fluid.

4. An installation as claimed in Claim 3, characterised by the following feature :

a) The steam gasifier (4) is heated by the cooling fluid of a nuclear reactor (13).

5. An installation as claimed in Claim 3, characterised by the following feature :

a) The steam gasifier (4) is heated by the product gas of the hydrogenating gasifier (3).

6. An installation as claimed in one or more of Claims 1-5, characterised by the following feature :

a) It includes a nuclear reactor (13) which serves to produce the steam which is supplied to the steam gasifier (4).

7. A process for the operation of an installation as claimed in Claim 5 or Claim 6, characterised by the following feature :

a) The heat required for equalising the heat balance within the installation when the material balance is already equalised, is produced by burning a part of the residual coke with separately supplied oxygen.

8. A process for the operation of an installation as claimed in Claim 5 or Claim 6, characterised by the following features :

a) The hydrogen which is required to equalise the material balance in the installation when the heat balance is already equalised, is produced from a part of the methane in a cracking furnace (17).

## Revendications

1. Installation pour gazéifier le charbon en méthane, comportant un gaséificateur d'hydrogénation (3) ; un gazéificateur à la vapeur d'eau (4) dans lequel on gazéifie le coke résiduaire provenant du gazéificateur d'hydrogénation ; un convertisseur (9) pour transformer, avec addition de vapeur d'eau et d'oxyde de carbone, l'oxyde de carbone produit dans le gazéificateur à la vapeur d'eau et une séparation du gaz (12) pour séparer le méthane, l'hydrogène et l'oxyde de carbone, caractérisée par la combinaison des moyens suivants :

a) le gazéificateur d'hydrogénation (3) est relié, par l'intermédiaire d'une conduite traversée par de l'hydrogène, aussi bien au gazéificateur à la vapeur d'eau (4) qu'au séparateur du gaz (12) ;

b) le gazéificateur d'hydrogénation (3) et le gazéificateur à la vapeur d'eau (4) sont disposés l'un au-dessus de l'autre ; le coke résiduaire s'écoulant directement du premier dans le second.

c) le gazéificateur à la vapeur d'eau (4) comporte plusieurs étages successifs de lits fluidisés, reliés entre eux.

2. Installation selon la revendication 1, caractérisée par le fait que le gazéificateur à la vapeur d'eau est chauffé, de manière connue, par combustion d'une partie de coke résiduaire avec de l'oxygène amené séparément.

3. Installation selon la revendication 1, caractérisé par le fait que le gazéificateur à la vapeur d'eau (4) est chauffé indirectement à l'aide d'un fluide chaud.

4. Installation selon la revendication 3, caractérisée par le fait que le gazéificateur à la vapeur d'eau (4) est chauffé avec un fluide de réfrigération d'un réacteur nucléaire (13).

5. Installation selon la revendication 3, caractérisée par le fait que le gazéificateur à la vapeur d'eau (4) est chauffé avec le gaz résiduaire du gazéificateur d'hydrogénation (3).

6. Installation selon l'une ou plusieurs des revendications 1 à 5, caractérisée par le fait qu'elle comprend un réacteur nucléaire (13) pour produire la vapeur d'eau amenée au gazéificateur à la vapeur d'eau (4).

7. Procédé pour la mise en œuvre d'une installation selon la revendication 5 ou 6, caractérisé par le fait que la chaleur nécessaire pour équilibrer le bilan thermique, lorsque le bilan en matériaux est équilibré, est produite par la combustion d'une partie du coke résiduaire avec de l'oxygène amené séparément.

8. Procédé pour la mise en œuvre d'une installation selon la revendication 5 ou 6, caractérisé par le fait que l'oxygène nécessaire pour équilibrer le bilan en matériaux, lorsque le bilan thermique est équilibré, est produit, dans un four de dissociation, à partir d'une partie de méthane.

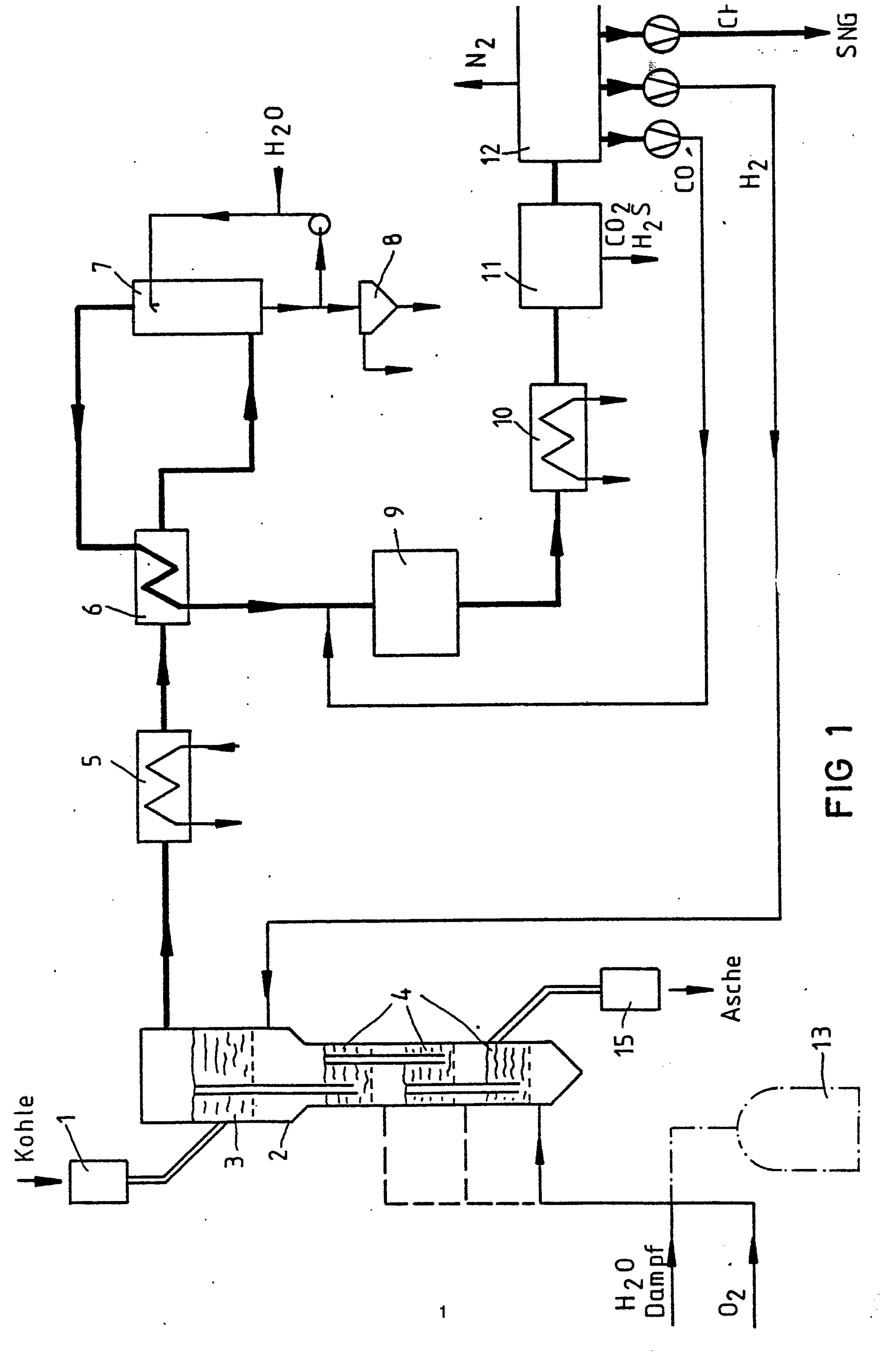

Kohle
1
2
3
4
5
6
7
8
9
10
11
12
13
15
$H_2O$
$N_2$
CH
SNG
CO
$H_2$
$CO_2$
$H_2S$
Asche
$H_2O$
Dampf
$O_2$


FIG 1

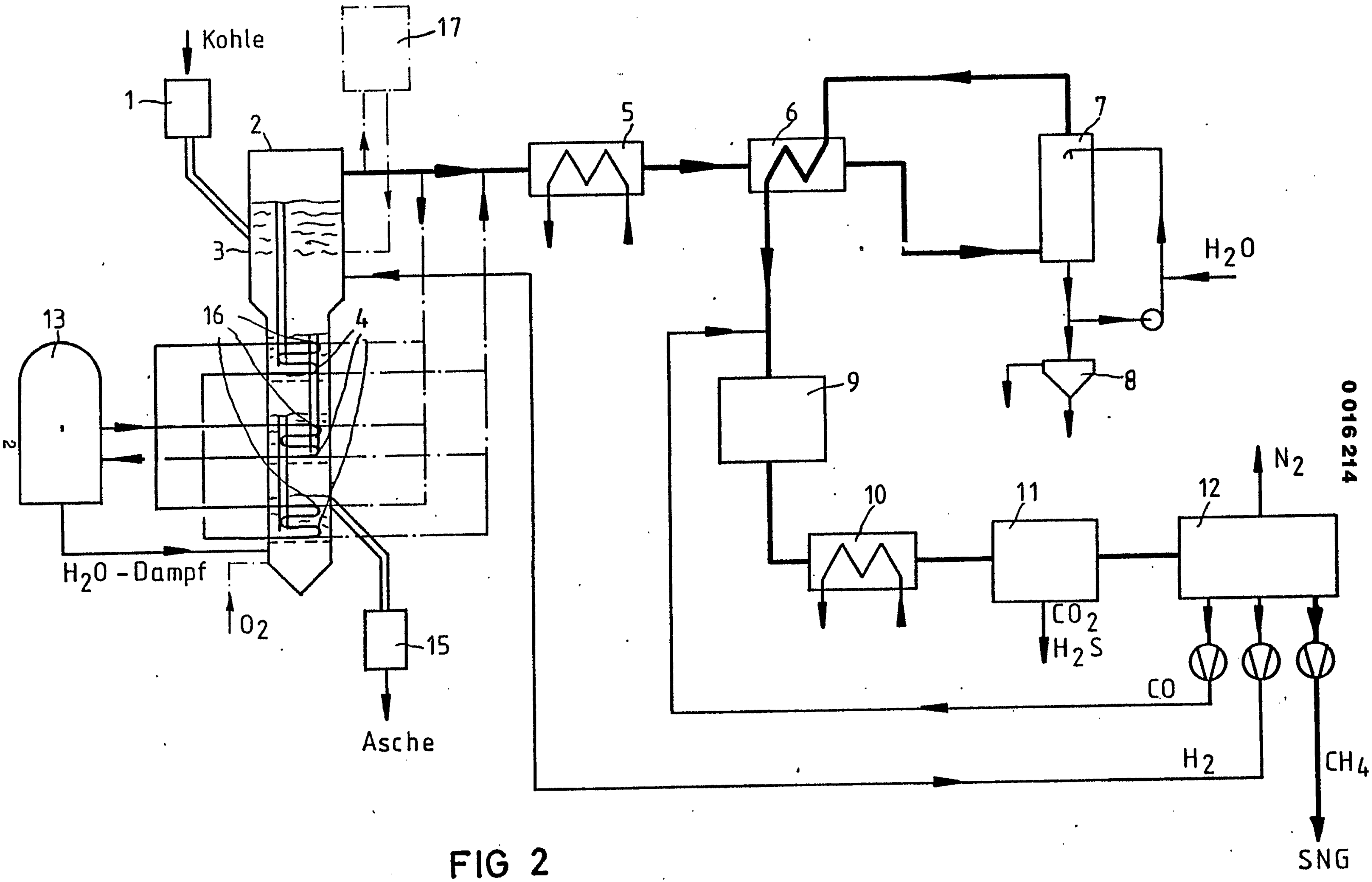
Kohle
1
2
3
17
5
6
7
H2O
8
9
10
11
12
N2
CO2
H2S
CO
H2
CH4
SNG
13
16
4
H2O - Dampf
O2
15
Asche

FIG 2